# EUROPÄISCHE PATENTSCHRIFT

(11) **EP 0 559 815 B1**
(45) Veröffentlichungstag und Bekanntmachung des Hinweises auf die Patenterteilung: **06.09.1995**
(21) Anmeldenummer: 92902580.7
(22) Anmeldetag: 22.11.1991
(51) Int. Cl.: C07D 513/04, A61K 31/425

(54) **OPTISCH AKTIVE THIAZOLOISOINDOLINON-DERIVATE MIT ANTIVIRALER WIRKUNG**
OPTICALLY ACTIVE THIAZOLOISOINDOLINONE DERIVATIVES WITH AN ANTIVIRAL ACTION
DERIVES OPTIQUEMENT ACTIFS DE THIAZOLO-ISOINDOLINONE A EFFET ANTIVIRAL

(30) Priorität: 27.11.1990 DE 4037674; 06.07.1991 DE 4122418
(43) Veröffentlichungstag der Anmeldung: 15.09.1993
(73) Patentinhaber: BOEHRINGER MANNHEIM GMBH, 68298 Mannheim (DE)
(72) Erfinder: ZILCH, Harald, D-6800 Mannheim 31 (DE); MERTENS, Alfred, D-6905 Schriesheim (DE); LEINERT, Herbert, D-6148 Heppenheim (DE); KÖNIG, Bernhard, D-8137 Berg 3 (DE); LESER, Ulrike, D-8000 München 70 (DE); SEIDEL, Hans, D-8132 Tutzing (DE)
(74) Vertreter: Köster, Reinhold, Dr.
(86) Internationale Anmeldenummer: EP9102205
(87) Internationale Veröffentlichungsnummer: WO9209606

(56) Entgegenhaltungen:
- CH-A- 469 733
- GB-A- 1 039 117
- JOURNAL OF THE CHEMICAL SOCIETY, PERKIN TRANSACTIONS 1. Nr. 7, Juli 1988, Letchworth, GB, Seiten 1837-1844; T. MCINALLY et al.: "Novel, base-induced fragmentation of Hantzsch-type 4-aryl-1,4-dihydropyridines"

## Beschreibung

Gegenstand der vorliegenden Erfindung sind optisch aktive Thiazoloisoindolinon-Derivate, diese enthaltende Arzneimittel sowie deren Verwendung zur Herstellung von Arzneimittel mit antiviraler Wirkung.

Die vorliegende Erfindung betrifft optisch aktive Verbindungen der allgemeinen Formel I und II 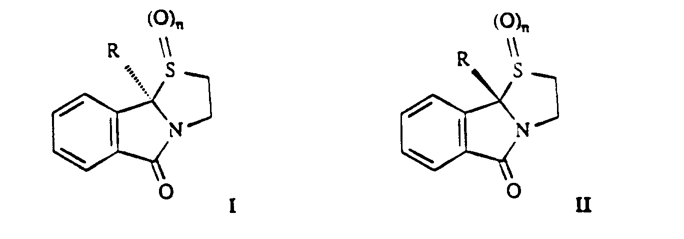
in denen
- R: ein Wasserstoffatom oder einen geradkettigen oder verzweigten, gesättigten oder ungesättigten aliphatischen Rest mit 1-7 Kohlenstoffatomen, der gegebenenfalls durch Phenyl substituiert sein kann, oder einen Phenylring, der gegebenenfalls ein- oder mehrfach durch C₁-C₄-Alkyl, C₁-C₄-Alkoxy, Hydroxy, Trifluormethyl, C₁-C₄-Alkylsulfonyl oder Halogen, wie Fluor, Chlor oder Brom, substituiert sein kann, bedeutet,
- n: für 0, 1 oder 2 steht,
sowie deren pharmakologisch verträgliche Salze und Tautomere.

In der deutschen Patentanmeldung P 40 35 809.7 sind die entsprechenden Racemate dieser Thiazoloisoindolinone beschrieben.

Es hat sich nun überraschenderweise gezeigt, daß die optisch aktiven Formen gegenüber den Racematen eine höhere pharmakologische Wirksamkeit besitzen. Insbesondere wurde gefunden, daß die neuen Verbindungen der Formel I und II in geringeren Dosen wirksam sind im Vergleich zu den Racematen, und sich durch eine bessere therapeutische Breite auszeichnen.

Im US-Patent 3,334,113 ist u.a. das 9b-Phenyl-2,3-dihydrothiazolo-[2,3-a]isoindol-5(9bH)-on als entzündungshemmendes und anticonvulsives Arzneimittel beschrieben.

Weitere Derivate des Thiazoloisoindolinons mit ähnlicher Wirkung und geringer Toxizität sind aus der schweizerischen Patentanmeldung CH-A-469,733 (R=Alkyl; n=0) und der belgischen Patentanmeldung BE-A-659,528 bzw. dem US-Patent 3,646,022 bekannt.

In U.S. 2,860,985 und der belgischen Patentanmeldung BE-A-564,592 werden Thiazoloisoindolinone zur Stabilisierung und als Kontrastmittel für photographische Emulsionen verwendet.

Die Synthese der racemischen Verbindungen ist außer in den genannten Patentanmeldungen auch in J. Org. Chem. 30, 1506 (1965) sowie J. Org. Chem. 34, 165 (1969) beschrieben.

Die Trennung der Racemate in die Enantiomere kann analytisch, semipräparativ und präparativ chromatographisch auf geeigneten optisch aktiven Phasen mit gängigen Elutionsmitteln durchgeführt werden. Als optisch aktive Phasen eignen sich beispielsweise optisch aktive Polyacrylamide oder Polymethacrylamide, z.T. auch an Kieselgel (z.B. ChiraSpher ^{(R)} von Merck, Chiralpak ^{(R)} OT/OP von Baker), Celluloseester/-carbamate (z.B. Chiracel ^{(R)} OB/OY von Baker/Daicel), Phasen auf Cyclodextrin- oder Kronenetherbasis (z.B. Crownpak ^{(R)} von Daicel) oder mikrokristallines Cellulosetriacetat (Merck).

Die Verbindungen der Formel I und II weisen eine ausgeprägte antivirale Wirkung auf, und eignen sich daher besonders gut zur Behandlung von viralen bzw. retro-viralen Infektionen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir^{R}, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431). Die Verbindungen der allgemeinen Formeln I und II besitzen diese Nachteile nicht. Sie wirken antiviral, ohne in pharmakologisch relevanten Dosen cytotoxisch zu sein.

Die Verbindungen der vorliegenden Erfindung eignen sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und -2, verursacht werden.

Besonders geeignet erscheinen die Verbindungen der Formel I und II zur Behandlung der klinischen Manifestationen der retroviralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Es konnte nun nachgewiesen werden, daß Verbindungen der allgemeinen Formel I und II die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773 1987).

Da ein sehr großer Bedarf an Chemotherapeutika besteht, die möglichst spezifisch mit retroviral bedingten Erkrankungen oder deren Symptomen interferieren, ohne die normal ablaufenden natürlichen Körperfunktionen zu beeinflussen, könnten die genannten Verbindungen vorteilhaft prophylaktisch oder therapeutisch bei der Behandlung von Krankheiten eingesetzt werden, bei denen eine retrovirale Infektion von pathophysiologischer, symptomatischer oder klinischer Relevanz ist.

In den Formeln I und II bedeutet R einen geradkettigen oder verzweigten, gesättigten oder ungesättigten aliphatischen Rest, insbesondere einen Alkylrest mit 1-7, vorzugsweise 1-4 Kohlenstoffatomen, wie z. B. Methyl, Ethyl oder Isopropyl. Der aliphatische Rest kann auch durch eine Phenylgruppe substituiert sein, so daß R einen Phenylalkylrest, wie z. B. Benzyl, darstellt. Als ungesättigte Reste kommen C₂-C₇-Alkenyl- oder C₂-C₇-Alkinylgruppen in Frage. R kann ferner einen Phenylring darstellen, der unsubstituiert oder ein- oder mehrfach substituiert sein kann. Bevorzugt ist der Phenylring ein- oder zweifach substituiert. Als Substituenten kommen beispielsweise die Methyl-, Ethyl-, Methoxy-, Ethoxy- oder Methylsulfonylgruppe in Frage.

Bevorzugt bedeutet R eine Phenylgruppe, die ein- bis dreifach substituiert sein kann durch die folgenden Reste: C₁-C₄-Alkyl, insbesondere Methyl, Ethyl, n-Propyl oder iso-Propyl; Halogen, insbesondere Fluor, Chlor oder Brom; Trifluormethyl; Hydroxy; C₁-C₄-Alkoxy, insbesondere Methoxy oder Ethoxy. Besonders bevorzugt sind solche Derivate, die in 3-Stellung des Phenylrings einen Substituenten tragen. Disubstituierte Phenylrest sind vorzugsweise die in 2,3-, 3,5-, 3,4-, 2,4- oder 2,5-Stellung substituierten Derivate.

Bevorzugt im Sinne der vorliegenden Erfindung sind optisch aktive Verbindungen der allgemeinen Formel I.

Arzneimittel, die mindestens eine Verbindung der Formel I oder II enthalten können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Vlskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Pharmakologisch verträgliche Salze der Verbindungen I und II sind Säureadditionssalze mit anorganischen oder organischen Säuren, wie z. B. Salzsäure, Bromwasserstoffsäure, Salpetersäure, Phosphorsäure, Essigsäure, Oxalsäure, Fumarsäure, Maleinsäure oder Bernsteinsäure.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Die Racemate der erfindungsgemäßen Verbindungen der allgemeinen Formeln I und II können nach Vorschriften der im Stand der Technik genannten Patentanmeldungen bzw. Literaturstellen hergestellt und auf geeigneten optisch aktiven Phasen chromatographisch getrennt werden.

Im Sinne der vorliegenden Erfindung kommen außer den in den Beispielen genannten Verbindungen und der durch Kombination aller in den Ansprüchen genannten Bedeutungen der Sustituenten die folgenden Verbindungen der Formel I in Frage:
1.) (R)-2,3-Dihydrothiazolo-[2,3-a]isoindol-5(9bH)-on
2.) (R)-9b-Methyl-2,3-dihydrothiazolo-[2,3-a]isoindol-5(9bH)-on
3.) (R)-9b-Phenyl-2,3-dihydrothiazolo-[2,3-a]isoindol-5(9bH)-on
4.) (R)-9b-(4-Methylphenyl)-2,3-dihydrothiazolo-[2,3-a]isoindol-5-(9bH)-on
5.) (R)-9b-(3-Methylphenyl)-2,3-dihydrothiazolo-[2,3-a]isoindol-5-(9bH)-on
6.) (R)-9b-(4-Ethylphenyl)-2,3-dihydrothiazolo-[2,3-a]isoindol-5-(9bH)-on
7.) (R)-9b-(2,4-Dimethylphenyl)-2,3-dihydrothiazolo-[2,3-a]isoindol-5(9bH)-on
8.) (R)-9b-(3,4-Dimethylphenyl)-2,3-dihydrothiazolo-[2,3-a]isoindol-5(9bH)-on
9.) (R)-9b-(2,5-Dimethylphenyl)-2,3-dihydrothiazolo-[2,3-a]isoindol-5(9bH)-on
10.) (R)-9b-(3-Trifluormethylphenyl)-2,3-dihydrothiazolo-[2,3-a]isoindol-5(9bH)-on
11.) (R)-9b-(4-Trifluormethylphenyl)-2,3-dihydrothiazolo-[2,3-a]isoindol-5(9bH)-on
12.) (R)-9b-(4-Hydroxyphenyl)-2,3-dihydrothiazolo-[2,3-a]isoindol-5(9bH)-on
13.) (R)-9b-(3-Hydroxyphenyl)-2,3-dihydrothiazolo-[2,3-a]isoindol-5(9bH)-on
14.) (R)-9b-(4-Ethoxyphenyl)-2,3-dihydrothiazolo-[2,3-a]isoindol-5-(9bH)-on
15.) (R)-9b-(3-Methoxyphenyl)-2,3-dihydrothiazolo-[2,3-a]isoindol-5(9bH)-on
16.) (R)-9b-(3-Fluorphenyl)-2,3-dihydrothiazolo-[2,3-a]isoindol-5-(9bH)-on
17.) (R)-9b-(4-Chlorphenyl)-2,3-dihydrothiazolo-[2,3-a]isoindol-5-(9bH)-on
18.) (R)-9b-(4-Bromphenyl)-2,3-dihydrothiazolo-[2,3-a]isoindol-5-(9bH)-on
19.) (R)-9b-(4-Methylsulfonylphenyl)-2,3-dihydrothiazolo-[2,3-a]isoindol-5(9bH)-on
20.) (R)-9b-Phenyl-2,3-dihydrothiazolo-[2,3-a]isoindol-5(9bH)-on-1-oxid
Weiterhin kommen die folgenden Verbindungen der Formel II in Frage:
1.) (S)-2,3-Dihydrothiazolo-[2,3-a]isoindol-5(9bH)-on
2.) (S)-9b-Methyl-2,3-dihydrothiazolo-[2,3-a]isoindol-5(9bH)-on
3.) (S)-9b-Phenyl-2,3-dihydrothiazolo-[2,3-a]isoindol-5(9bH)-on
4.) (S)-9b-(4-Methylphenyl)-2,3-dihydrothiazolo-[2,3-a]isoindol-5-(9bH)-on
5.) (S)-9b-(3-Methylphenyl)-2,3-dihydrothiazolo-+ [2,3-a]isoindol-5-(9bH)-on
6.) (S)-9b-(4-Ethylphenyl)-2,3-dihydrothiazolo-[2,3-a]isoindol-5-(9bH)-on
7.) (S)-9b-(2,4-Dimethylphenyl)-2,3-dihydrothiazolo-[2,3-a]isoindol-5(9bH)-on
8.) (S)-9b-(3,4-Dimethylphenyl)-2,3-dihydrothiazolo-[2,3-a]isoindol-5(9bH)-on
9.) (S)-9b-(2,5-Dimethylphenyl)-2,3-dihydrothiazolo-[2,3-a]isoindol-5(9bH)-on
10.) (S)-9b-(3-Trifluormethylphenyl)-2,3-dihydrothiazolo-[2,3-a]isoindol-5(9bH)-on
11.) (S)-9b-(4-Trifluormethylphenyl)-2,3-dihydrothiazolo-[2,3-a]isoindol-5(9bH)-on
12.) (S)-9b-(4-Hydroxyphenyl)-2,3-dihydrothiazolo-[2,3-a]isoindol-5(9bH)-on
13.) (S)-9b-(3-Hydroxyphenyl)-2,3-dihydrothiazolo-[2,3-a]isoindol-5(9bH)-on
14.) (S)-9b-(4-Ethoxyphenyl)-2,3-dihydrothiazolo-[2,3-a]isoindol-5-(9bH)-on
15.) (S)-9b-(3-Methoxyphenyl)-2,3-dihydrothiazolo-[2,3-a]isoindol-5(9bH)-on
16.) (S)-9b-(3-Fluorphenyl)-2,3-dihydrothiazolo-[2,3-a]isoindol-5-(9bH)-on
17.) (S)-9b-(4-Chlorphenyl)-2,3-dihydrothiazolo-[2,3-a]isoindol-5-(9bH)-on
18.) (S)-9b-(4-Bromphenyl)-2,3-dihydrothiazolo-[2,3-a]isoindol-5-(9bH)-on
19.) (S)-9b-(4-Methylsulfonylphenyl)-2,3-dihydrothiazolo-[2,3-a]isoindol-5(9bH)-on
20.) (S)-9b-Phenyl-2,3-dihydrothiazolo-[2,3-a]isoindol-5(9bH)-on-1-oxid

### Beispiel 1

### Enantiomerentrennung von rac-9b-Phenyl-2,3-dihydrothiazolo-[2,3-a]isoindol-5(9bH)-on an Cellulosetriacetat

Für die Trennung der Antipoden wurden 500 mg des Racemats in 10 ml Ethanol gelöst, auf eine Säule mit 50 mm Innendurchmesser und 300 mm Länge (entsprechend 250 g Cellulosetriacetat, 15-25 µm Korngröße, Merck 16326) aufgegeben und mit Ethanol eluiert (Fluß 7.5 ml/min, ca. 1.5 bar).
- Peak I:: UV-Detektion bei 254 nm, Run Time: 130 min;
[α]_{D}²⁰ = 342°, Schmp. 99-100° C;
absolute Konfiguration: (S)-Form
- Peak II:: UV-Detektion bei 254 nm; Run Time: 165 min;
[α]D²⁰ = +344°; Schmp. 99-100° C;
absolute Konfiguration: (R)-Form

Die Enantiomeren wurden aus Ethanol umkristallisiert.

Analog zu Beispiel 1 wurden folgende Racemate an Cellulosetriacetat getrennt:
1.1 rac-9b-(4-Methylphenyl)-2,3-dihydrothiazolo-[2,3-a]-isoindol-5(9bH)-on
1.2 rac-9b-(3-Methylphenyl)-2,3-dihydrothiazolo-[2,3-a]-isoindol-5(9bH)-on
1.3 rac-9b-(3,4-Dimethylphenyl)-2,3-dihydrothiazolo-[2,3-a]-isoindol-5(9bH)-on
1.4 rac-9b-(3,4-Dichlorphenyl)-2,3-dihydrothiazolo-[2,3-a]-isoindol-5(9bH)-on
1.5 rac-9b-(2,3-Dimethylphenyl)-2,3-dihydrothiazolo-[2,3-a]-isoindol-5(9bH)-on
1.6 rac-9b-(3-Chlorphenyl)-2,3-dihydrothiazolo-[2,3-a]-isoindol-5(9bH)-on
   Methanol/20 % H₂O als Eluens; Umkristallisation aus Ethanol
1.7 rac-9b-(3,5-Dimethylphenyl)-2,3-dihydrothiazolo-[2,3-a]-isoindol-5(9bH)-on
   Methanol/20 % H₂O als Eluens, Umkristallisation aus Ethanol

| Bsp. | Run Time Peak I | Run Time Peak II | [α]_{D}²⁰ | Schmp. [°C] |
|---|---|---|---|---|
| 1.1 | 105 | | -275 | Öl |
| | | 140 | +270 | Öl |
| 1.2 | 135 | | -294 | 84-88 |
| | | 190 | +288 | 84-88 |
| 1.3 | 130 | | -241 | Öl |
| | | 180 | +231 | Öl |
| 1.4 | 135 | | -242 | Öl |
| | | 185 | +231 | Öl |
| 1.5 | 120 | | -239 | Öl |
| | | 175 | +235 | Öl |
| 1.6 | 95 | 130 | -303 | 107-109 |
| | | | +307 | 107-109 |
| 1.7 | 105 | 155 | -316 | 170-174 |
| | | | +319 | 170-174 |

### Beispiel 2

### Enantiomerentrennung von rac-9b-Phenyl-2,3-dihydro-thiazolo-[2,3-a]isoindol-5(9bH)-on an Chiracel ^{(R)} OB

Für die Trennung im semipräparativen Meßstab wurden 2 ml einer kalt gesättigten Lösung des Racemats in einer Mischung aus Isopropanol/Hexan 30:70 auf die optisch aktive Phase gegeben und mit dem genannten Lösungsmittelgemisch eluiert (Chiracel ^{(R)}OB von Baker/Daicel, 10 mm Innendurchmesser, 300 mm Länge, Fluß 1 ml/min, 10 µm Korngröße). RunTime 12 bzw. 17 min.

Die Daten waren mit denen der isolierten Enantiomeren von Bsp. 1 vergleichbar.

### Beispiel 3

### Hemmung der Reversen Transkriptase (RT) durch (R)- und (S)-9b-Phenyl-2,3-dihydrothiazolo-[2,3-a]isoindol-5(9bH)-on

Das Screeningtestsystem beinhaltet die gereinigte RT aus HIV-1, die durch gentechnologische Methoden in E. coli exprimiert wurde, sowie die Komponenten des Initiationskomplexes, wie die in-vitro-Transkripte des HIV-LTR's mit der benachbarten Primer Binding Site als Template und einem zur Primer Binding Site komplementären 18mer Oligonukleotid als Primer. Gemessen wurde der [³H]-Thymidin-5'-triphosphat-Einbau durch Auszählen im β-Counter.

| Ergebnisse: | |
|---|---|
| Substanz | Hemmung der HIV-RT IC₅₀[M] |
| 3'-Azido-3'-desoxythymidin-5'-triphosphat [AZT-TP] | 6.0 x 10⁻⁶ |
| (R)-9b-Phenyl-2,3-dihydrothiazolo-[2,3-a]isoindol-5(9bH)-on | 0.4 x 10⁻⁶ |
| (S)-9b-Phenyl-2,3-dihydrothiazolo-[2,3,-a]isoindol-5(9bH)-on | 26.5 x 10⁻⁶ |

## Patentansprüche

1. Optisch aktive Thiazoloisoindolinone der allgemeinem Formel I oder II 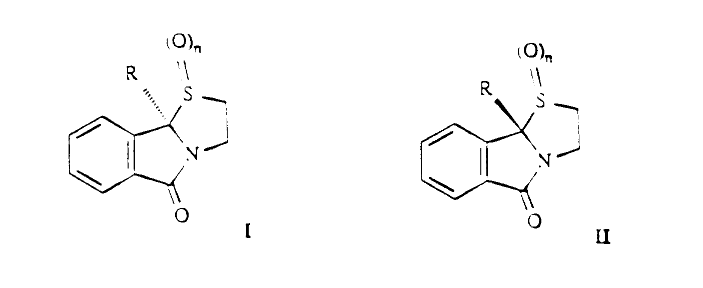 in denen
R ein Wasserstoffatom oder einen geradkettigen oder verzweigten, C₁-C₇-Alkyl-, C₂-C₇-Alkenyl- oder C₂-C₇-Alkinylrest, der gegebenenfalls durch Phenyl substituiert sein kann, oder einen Phenylring, der gegebenenfalls ein- oder mehrfach durch C₁-C₄-Alkyl, C₁-C₄-Alkoxy, Hydroxy, Trifluormethyl, C₁-C₄-Alkylsulfonyl oder Halogen, wie Fluor, Chlor oder Brom substituiert sein kann, bedeutet,
n für 0, 1 oder 2 steht,
sowie deren pharmakologisch verträgliche Salze und Tautomere.

2. Optisch aktive Thiazoloisoindolinone gemäß Anspruch 1, dadurch gekennzeichnet, daß R ein Wasserstoffatom oder einen gesättigten oder ungesättigten, geradkettigen oder verzweigten aliphatischen Rest mit 1-7 C-Atomen bedeutet.

3. Optisch aktive Thiazoloisoindolinone gemäß Anspruch 1, dadurch gekennzeichnet, daß R einen C₁-C₇-Alkylrest bedeutet, der durch eine Phenylgruppe substituiert sein kann.

4. Optisch aktive Thiazoloisoindolinone gemäß Anspruch 1, dadurch gekennzeichnet, daß R eine Phenylgruppe bedeutet, die unsubstituiert oder ein- bis dreifach substituiert ist durch C₁-C₄-Alkyl, Halogen oder Hydroxy.

5. Optisch aktive Thiazoloisoindolinone gemäß Anspruch 1 ausgewählt aus der Gruppe der folgenden Verbindungen:
9b-Phenyl-2,3-dihydrothiazolo-[2,3-a]isoindol-5(9bH)-on
9b-(4-Methylphenyl)-2,3-dihydrothiazolo-[2,3-a]-isoindol-5(9bH)-on
9b-(3-Methylphenyl)-2,3-dihydrothiazolo-[2,3-a]-isoindol-5(9bH)-on
9b-(3,4-Dimethylphenyl)-2,3-dihydrothiazolo-[2,3-a]-isoindol-5(9bH)-on
9b-(3,4-Dichlorphenyl)-2,3-dihydrothiazolo-[2,3-a]-isoindol-5(9bH)-on
9b-(2,3-Dimethylphenyl)-2,3-dihydrothiazolo-[2,3-a]-isoindol-5(9bH)-on
9b-(3-Chlorphenyl)-2,3-dihydrothiazolo-[2,3-a]-isoindol-5(9bH)-on
9b-(3,5-Dimethylphenyl)-2,3-dihydrothiazolo-[2,3-a]-isoindol-5(9bH)-on

6. Arzneimittel enthaltend ausschließlich optisch aktive Verbindungen der Formel I (R-Isomere) gemäß einem der Ansprüche 1 - 5, sowie pharmakologisch verträgliche Träger- oder Hilfsstoffe.

7. Arzneimittel enthaltend ausschließlich optisch aktive Verbindungen der Formel II (S-Isomere) gemäß einem der Ansprüche 1 - 5, sowie pharmakologisch verträgliche Träger- oder Hilfsstoffe.

8. Verwendung von Verbindungen der Formel I oder II gemäß einem der Ansprüche 1 - 5 zur Herstellung von Arzneimitteln mit antiviraler Wirkung.

9. Verfahren zur Herstellung von optisch aktiven Thiazoloisoindolinone gemäß einem der Ansprüche 1 - 5, dadurch gekennzeichnet, daß man die entsprechenden Racemate durch chromatographische Methoden in die optisch aktiven Verbindungen auftrennt.

## Claims

1. Optically-active thiazoloisoindolinones of the general formulae I or II 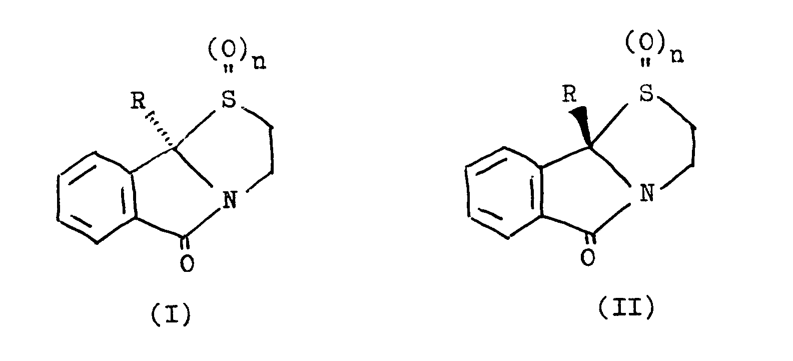 in which R signifies a hydrogen atom or a straight-chained or branched C₁-C₇-alkyl, C₂-C₇-alkenyl or C₂-C₇-alkynyl radical which can possible be substituted by phenyl, or a phenyl ring which can possibly be substituted one or more times by C₁-C₄-alkyl, C₁-C₄-alkoxy, hydroxyl, trifluoromethyl, C₁-C₄-alkylsulphonyl or halogen, such as fluorine, chlorine or bromine and n stands for 0, 1 or 2, as well as their pharmacologically compatible salts and tautomers.

2. Optically-active thiazoloisoindolinones according to claim 1, characterised in that R signifies a hydrogen atom or a saturated or unsaturated, straight-chained or branched aliphatic radical with 1 - 7 C-atoms.

3. Optically-active thiazoloisoindolinones according to claim 1, characterised in that R signifies a C₁-C₇-alkyl radical which can be substituted by a phenyl group.

4. Optically-active thiazoloisoindolinones according to claim 1, characterised in that R signifies a phenyl group which is unsubstituted or substituted one to three times by C₁-C₄-alkyl, halogen or hydroxyl.

5. Optically-active thiazoloisoindolinones according to claim 1 selected from the group of the following compounds:
9b-phenyl-2,3-dihydrothiazolo-[2,3-a]-isoindol-5(9bH)-one
9b-(4-methylphenyl)-2,3-dihydrothiazolo-[2,3-a]-isoindol-5(9bH)-one
9b-(3-methylphenyl)-2,3-dihydrothiazolo-[2,3-a]-isoindol-5(9bH)-one
9b-(3,4-dimethylphenyl)-2,3-dihydrothiazolo-[2,3-a]-isoindol-5(9bH)-one
9b-(3,4-dichlorophenyl)-2,3-dihydrothiazolo-[2,3-a]-isoindol-5(9bH)-one
9b-(2,3-dimethylphenyl)-2,3-dihydrothiazolo-[2,3-a]-isoindol-5(9bH)-one
9b-(3-chlorophenyl)-2,3-dihydrothiazolo-[2,3-a]-isoindol-5(9bH)-one
9b-(3,5-dimethylphenyl)-2,3-dihydrothiazolo-[2,3-a]-isoindol-5(9bH)-one.

6. Medicaments containing exclusively optically-active compounds of the formula I (R-isomers) according to one of claims 1 - 5, as well as pharmaceutically compatible carrier or adjuvant materials.

7. Medicaments containing exclusively optically-active compounds of the formula II (S-isomers) according to one of claims 1 - 5, as well as pharmaceutically compatible carrier or adjuvant materials.

8. Use of compounds of the formula I or II according to one of claims 1 - 5 for the preparation of medicaments with anti-viral action.

9. Process for the preparation of optically-active thiazoloisoindolinones according to one of claims 1 - 5, characterised in that one separates the corresponding racemates into the optically-active compounds by chromatographic methods.

## Revendications

1. Thiazoloisoindolinones optiquement actives, de formule générale I ou II 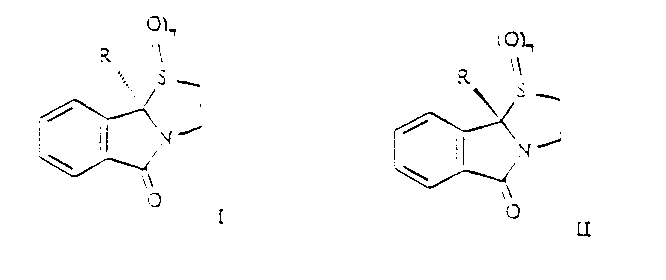 où
R représente un atome d'hydrogène ou un reste alkyle en C₁-C₇, alcényle en C₂-C₇ ou alcinyle en C₂-C₇, à chaîne linéaire ou ramifiée, qui peut être éventuellement substitué par un groupe phényle, ou un noyau phényle qui peut être substitué éventuellement une ou plusieurs fois par un reste alkyle en C₁-C₄, alcoxy en C₁-C₄, hydroxy, trifluorométhyle, alkyle(C₁-C₄)sulfonyle ou un halogéne tel que le fluor, le chlore ou le brome,
n vaut 0, 1 ou 2,
ainsi que leurs sels et tautomères pharmacologiquement acceptables.

2. Thiazoloisoindolinones optiquement actives selon la revendication 1, caractérisées en ce que R représente un atome d'hydrogène ou un reste aliphatique saturé ou insaturé, à chaîne linéaire ou ramifiée, ayant 1-7 atomes de carbone.

3. Thiazoloisoindolinones optiquement actives selon la revendication 1, caractérisées en ce que R représente un reste alkyle en C₁-C₇ qui peut être substitué par un groupe phényle.

4. Thiazoloisoindolinones optiquement actives selon la revendication 1, caractérisées en ce que R représente un groupe phényle, qui n'est pas substitué ou qui est substitué une à trois fois par un groupe alkyle en C₁-C₄, halogéno ou hydroxy.

5. Thiazoloisoindolinones optiquement actives selon la revendication 1, choisies dans le groupe des composés suivants :
9b-phényl-2,3-dihydrothiazolo-[2,3a]-isoindol-5(9bH)-one
9b-(4-méthylphényl)-2,3-dihydrothiazolo-[2,3-a]isoindol-5(9bH)-one
9b-(3-méthylphényl)-2,3-dihydrothiazolo-[2,3-a]isoindol-5(9bH)-one
9b-(3,4-diméthylphényl)-2,3-dihydrothiazolo-[2,3-a]isoindol-5(9bH)-one
9b-(3,4-dichlorophényl)-2,3-dihydrothiazolo-[2,3-a]isoindol-5(9bH)-one
9b-(2,3-diméthylphényl)-2,3-dihydrothiazolo-[2,3-a]isoindol-5(9bH)-one
9b-(3-chlorophényl)-2,3-dihydrothiazolo-[2,3-a]isoindol-5(9bH)-one
9b-(3,5-diméthylphényl)-2,3-dihydrothiazolo-[2,3-a]isoindol-5(9bH)-one

6. Médicament contenant exclusivement des composés de formule I optiquement actifs (isomère R) selon l'une des revendications 1-5, ainsi que des véhicules et adjuvants pharmacologiquement acceptables.

7. Médicament contenant exclusivement des composés de formule II optiquement actifs (isomère S) selon l'une des revendications 1-5, ainsi que des véhicules et adjuvants pharmacologiquement acceptables.

8. Utilisation de composés de formule I ou II selon l'une des revendications 1-5, pour la préparation de médicaments ayant une activité antivirale.

9. Procédé de préparation de thiazoloindolinones optiquement actives selon l'une quelconque des revendications 1-5, caractérisé en ce que l'on sépare le racémate correspondant par des méthodes chromatographiques en les composés optiquement actifs.
